# EUROPEAN PATENT APPLICATION

(11) **EP 1 152 063 A1**
(43) Date of publication of application: **07.11.2001**
(21) Application number: 00109436.6
(22) Date of filing: 03.05.2000
(51) Int. Cl.: C12Q 1/70, C12Q 1/68

(54) **Method of diagnosing HBV infection stages**

(71) Applicant: Deutsches Krebsforschungszentrum Stiftung des öffentlichen Rechts, 69120 Heidelberg (DE)
(72) Inventor: Schröder, Klaus Hobe, Prof., 69124 Heidelberg (DE); Koike, Katsuro, Prof. JP Found.for Cancer Research, Tokyo 180-8455 (JP)
(74) Representative: Schüssler, Andrea, Dr.

(57) **Abstract**

The present invention concerns a method for the diagnosis of HBV infection stages which comprises the identification of full-length HBV transcripts (I) and truncated HBV transcripts (II), preferably transcripts comprising the X-region of HBV (HBx RNA), in a serum sample wherein the ratio of I:II is indicative of a particular infection stage. A ratio of full-length HBV transcripts (I) : truncated HBV transcripts (II) > 1 is indicative of a replicative HBV infection stage, whereas a ratio of full-length HBV transcripts (I) : truncated HBV transcripts (II) < 1 is indicative of a chronic HBV infection stage and, in addition, indicative of a risk to develop HCC.

## Description

The present invention relates to a method for the diagnosis of Hepatitis B virus (HBV) infection stages which comprises the identification of full-length HBV transcripts (I) and truncated HBV transcripts (II), preferably transcripts comprising the X-region of HBV (HBx RNA), in a serum sample wherein the ratio of I:II is indicative of a particular infection stage. In a preferred embodiment, the identification of said transcripts is carried out by RT/PCR. A ratio of full-length HBV transcripts (I) : truncated HBV transcripts (II) > 1 is indicative of a replicative HBV infection stage, whereas a ratio of full-length HBV transcripts (I) : truncated HBV transcripts (II) < 1 is indicative of a chronic HBV infection stage and, in addition, indicative of a risk to develop hepatocellular carcinoma.

The prevalence of chronic infection with hepatitis B virus (HBV) is estimated to be about 6 % of total population of the earth, varying greatly among different geographical regions. It is high ( ≥ 8 %) in some areas such as China, Southeast Asia and Sub-Sahara Africa. In these regions, about 60% of individuals have a history of an infection. In many cases, infection is self-limited and asymptomatic, and the virus is believed to be eliminated by the immune system leaving no detectable serological traces. In other cases infection becomes chronic as conventionally evaluated by serodetection for hepatitis B surface antigen (HBs). The risk for individuals exposed to the virus to become chronic carriers correlates inversely with age, being high (up to 90%) for infants and low (3-8%) among the adolescent or adult patients. The chronic infection may be asymptomatic for many years or result in only slight liver damages; in a certain number of cases, it leads to progressive liver diseases, namely chronic hepatitis and cirrhosis. In both cases an increase in the relative risk to develop hepatocellular carcinoma (HCC) by the factors of 100 and of 480, respectively, was observed. Thus, the chronic HBs-carrier state, whether causing the apparent liver diseases or not, has been linked to development of advanced hepatic preneoplastic lesions and HCC which is one of the most prevalent human cancers in the endemic areas, being the third most prevalent malignancy in males. More than 437,000 new cases of HCC are found each year, their 5-year survival rate being estimated to be below 3%. HBV is considered to be one of the main causative factors of liver cancer, possibly accounting for 60% of cases worldwide and about 70% of cases in endemic areas. Vaccination programs in several areas have been successful in protecting most of young individuals from being infected and even in reducing the risk for liver cancer. For those already infected it remains an important task to develop more discriminative screening procedures for addressing infection stages which may be critical for the development of HCC.

Four overlapping open reading frames on viral DNA, namely PreC/C, polymerase, PreS1/PreS2/S, and X, are transcribed into genomic RNA molecules of about 3,500 bp and into subgenomic transcripts ranging from 2,400 bp to 700 bp. One of the discernible genomic RNA molecules, designated as pregenome, serves as an intermediate during the replication of viral DNA. The translation product of the smallest transcripts, HBV x protein (HBx), has been implicated as a hepatocarcinogenic factor. Properties assigned to HBx include the stimulation of transcription and signal transduction, interference with DNA repair, induction of apoptosis and the induction of malignant transformation both in vitro and in vivo. Although the mechanism of HBV-associated hepatocarcinogenesis is not unambiguously established, integration of the viral DNA into chromosomes of hepatocytes, preferential preservation of HBx open reading frame sequences and selective albeit rare expression of HBx in preneoplastic and neoplastic human hepatocytes may be critical events.

During replication of HBV, all viral transcripts mature at a unique polyadenylation [poly(A)] signal downstream of the HBx open reading frame on viral DNA, a TATAAA motif at position 1789 (Cattaneo et al., Nature (London) 305 (1983), 336-338; Pourcel et al., J. Virol. 42 (1983), 100-105; Simonsen et al., Mol. Cell. Biol. 3 (1983), 2250-2258). Collectively these transcripts are addressed in the present invention as full-length transcripts. For most chromosomally integrated DNA, generation of full-length transcripts is unlikely. Truncations on the DNA level affecting the 3' end region of the HBx open reading frame are frequent (Hilger et al., J. Virol. 65 (1991), 4284-4291; Matsubara et al., Mol. Biol. Med. 7 (1990), 243-260; Hsia et al., Biochem. Biophys. Res. Commun. 241 (1997), 726-729; Poussin et al., In. J. Cancer 80 (1999), 497-505), leading to fusion of HBx sequences to adjacent cellular sequences and giving rise to some HBx/cell hybrid transcripts which have the capacity to direct the synthesis of functionally active HBx/cell fusion proteins.

A type of viral RNA, addressed as truncated, uses a cryptic poly(A) signal, a CAUAAA motif within HBx open reading frame (position 1661). It has been described to be present in HCC tissues and the surrounding liver parenchyma (Hilger et al., J. Virol. 65 (1991), 4284-4291). Truncated transcripts most likely are transcribed from chromosomally integrated HBV DNA which is present in chronically infected liver, its accumulation probably starting with the primary infection. The methods used so far for diagnosing an HBV infection are mainly based on serodiagnostic assays, however, unfortunately, there are HBV infection stages which escape conventional serodiagnosis, particularly chronical infection stages, i.e. non-replicative, cryptic infection stages. Although, this infection stage can theoretically be assayed by taking liver biopsies, it has to be stressed that a biopsie is not necessarily representative for the overall situation in the liver.

Thus, the technical problem underlying the present invention is to provide a method for the diagnosis of HBV infection stages (and diseases associated with these infection stages) which, so far, could not be analysed and which does not require a liver biopsy.

The solution to the above technical problem has been achieved by providing the embodiments characterized in the claims. It has been found that by the identification of individual HBV transcript types released from hepatocytes and circulating in sera a safe and convenient diagnosis of particular infection stages, particularly infection stages which were so far unrecognized, e.g. cryptic HBV infections, can be performed. During the experiments leading to this invention full-length and truncated transcripts of HBV were identified in sera of chronic carriers by using an RT/PCR assay involving anchored oligo(dT) primers. Presence of full-length RNA was correlated with seropositivity for hepatitis B e antigen and for viral DNA, seromarkers indicative for replicative processes. Truncated RNA was independent of these markers. Similarly there was no correlation between the levels of truncated RNA in the serum and the apparent liver damage indicated by transaminase levels. Overall, age-dependent representations of full-length and truncated RNA were different, with the former decreasing progressively to levels below detection limit and the latter reaching high levels during the first two decade of life and remaining elevated throughout the following decades. Truncated RNA and viral transcripts polyadenylated at neither of the two addition sites were detected even in the absence of any other conventional viral marker including viral DNA.

Thus, the present invention relates to a method for the diagnosis of HBV infection stages which comprises the identification of full-length HBV transcripts (I) and truncated HBV transcripts (II) in a serum sample, wherein the ratio of the amounts of I:II is indicative of a particular infection stage.

The term "full-length HBV transcripts" as used herein relates to transcripts which allow the translation of the complete protein. The term "truncated HBV transcripts" as used herein relates to shortened versions of the full-length transcripts which carry truncations at the 3'end, e.g. due to the usage of a cryptic poly(A) signal.

Methods for the preparation of serum samples and for the isolation and purification of nucleic acids, if required, are well known to the person skilled in the art. The identification of the full-length transcripts and truncated transcripts, the determination of length and concentration can be carried out by several standard methods including Northern blot analysis, RNAse protection, PCR, RT/PCR, LCR etc. Suitable probes and/or primers can be selected by the person skilled in the art according to the published sequences of the various genes of the HBV genome, i.e., PreC/C, polymerase, PreS1/PreS2/S, and X. The fact that there is a co-terminal maturation of HBV transcripts initiated at different sites (Cattaneo et al., Nature (London), 305 (1983), S. 336-338). implies the possibility that the 3'end structures of full length and truncated transcripts exist for all viral transcripts mentioned. However, only the HBx reading frame would be affected by polyadenylation mediated truncation. When using analysis methods like Northern blot the probes can be detectably labeled, for example, with a radioisotope, a fluorescent compound, a bioluminscent compound, a chemiluminescent compound, a metal chelator, or an enzyme. Those of ordinary skill in the art will know of other suitable labels for binding to the probes.

RNAse protection assays can also be used to detect the presence of full-length or truncated transcripts. One illustrative RNAse protection probe is an in vitro synthesized RNA comprised of sequences complementary to full-length transcript sequences and additional, non-complementary sequences. The latter sequences are included to distinguish the full-length probe from the fragment of the probe that results from a positive result in the assay: in a positive assay, the complementary sequences of the probe are protected from RNase digestion, because they are hybridized to full-length transcript sequences. The non-complementary sequences are digested away from the probe in the presence of RNase and target complementary nucleic acid.

In a preferred embodiment of the diagnostic method of the invention a ratio of full-length HBV transcripts (I) : truncated HBV transcripts (II) > 1 is indicative of a highly replicative HBV infection stage. This judgement is based on the fact that the HBV genome replicates via an RNA intermediate and that truncated RNA lacks sequences required for the initiation of minus strand DNA synthesis. The term "a ratio of full-length HBV transcripts (I) : truncated HBV transcripts (II) > 1" as used herein also comprises the situation where no truncated HBV transcripts can be detected.

In an alternative preferred embodiment of the diagnostic method of the invention a ratio of full-length HBV transcripts (I) : truncated HBV transcripts (II) < 1 is indicative of a late chronic HBV infection stage. An increase in the fraction of truncated RNA during the course of the chronic infection has been found for viral RNA extracted from tissue material and for RNA extracted from sera (Kairat et al., Intervirology 42 (1999), S. 228-237). The term "a ratio of full-length HBV transcripts (I) : truncated HBV transcripts (I) < 1" as used herein also comprises the situation where no full-length HBV transcripts can be detected, i.e. replication competent RNA is absent. Thereby occult stages of the HBV infection are recognized.

In a further alternative preferred embodiment of the diagnostic method of the invention a ratio of full-length HBV transcripts (I) : truncated HBV transcripts (II) < 1 is indicative of an elevated risk to develop HCC. This is based on the assumption that HCC represents a late of a chronic infection and the above observation that the fraction of truncated RNA increases with time.

In a more preferred embodiment of the diagnostic method of the present invention the HBV transcripts are transcripts comprising the X-region of HBV (HBx RNA). Probes and primers for determining the length and/or concentration of said transcripts can be designed on the basis of published sequences; see, e.g. Cattaneo et al., Nature (London) 305 (1983), 336-338; Pourcel et al., J. Virol. 42 (1983), 100-105; Simonsen et al., Mol. Cell. Biol. 3 (1983), 2250-2258; Hilger et al., J. Virol. 65 (1991), 4284-4291; Matsubara et al., Mol. Biol. Med. 7 (1990), 243-260; Hsia et al., Biochem. Biophys. Res. Commun. 241 (1997), 726-729; Poussin et al., In. J. Cancer 80 (1999), 497-505; Luber et al., Oncogene 12 (1996), 1597-1608; Rakotomahanina et al., Oncogene 9 (1994), 2613-2621; Schluter et al., Oncogene 9 (1994), 1-10; Takada and Koike, PNAS (USA) 87 (1990), 5628-5632; Wollersheim et al., Oncogene 3 (1988), 545-552.

In a still more preferred embodiment of the diagnostic method of the present invention the identification of full-length HBV transcripts (I) and truncated HBV transcripts (II) is carried out by RT/PCR using downstream primer(s) allowing to discriminate between full-length HBV transcripts (I) and truncated HBV transcripts (II), e.g. as described in Examples 1 and 2, below. General methods for carrying out RT/PCR are well known to the person skilled in the art and e.g. described in Cacciola et al., N Engl. J. Med. (1999), 341: 22-26. The person skilled in the art can also select primers which can discriminate between full-length HBV transcripts and truncated transcripts and/or which lead to the synthesis of amplification products with lengthes reflecting the full-length or truncated status of the transcripts. For example, full-length HBV transcripts and truncated HBV transcripts can be amplified in one test tube using only two different primers: one upstream primer (annealing to both kinds of transcripts) and one dowstream primer (also annealing to both kinds of transcripts), leading to the synthesis of amplification products with different lengthes corresponding to the RNA template (full-length vs. truncated.). Finally, the person skilled in the art is aware that controll RT/PCR reactions have to be included in order to demonstrate specifity of the reaction, to avoid the generation of false-positive results, to prove the integrity of the cellular RNA contained in the serum sample etc.

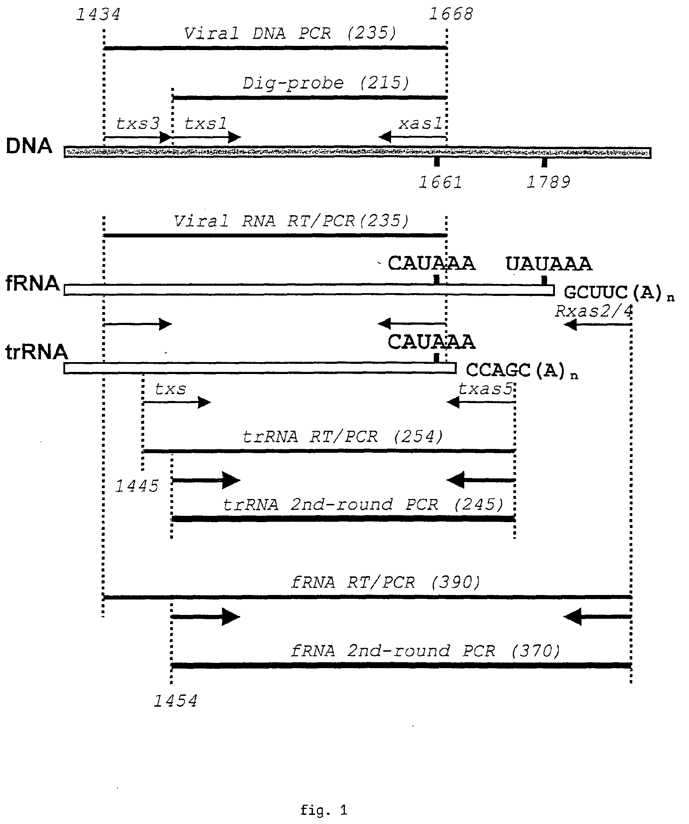

Particularly preferred is RT/PCR using as downstream primers oligo(dT)anchored primers, which can, e.g., distinguish between transcripts using different poly(A) signals, e.g. the native signal and a cryptic signal, respectively; see Figure 1 for further explanation. These primers are RNA specific and optionally present DNA will not be amplified. Most preferred are downstream oligo(dT)anchored primers comprising the nucleotide sequence (T)₁₅ GCT GG, (T)₁₅ GAA GC or (T)₁₅ AGC TC. These primers may be provided in form of a kit which further comprises conventional buffers and reagents for carrying out RT/PCR, like the HCV Genotyping Kit (Sorin - Biomedica, Saluggia, Italy) or the AMPLICOR HBV MONITOR test (Roche). In these tests the specific complementary primers can be replaced by the above identified primers of the present invention.

In a further preferred embodiment of the diagnostic method of the invention the identification of full-length HBV transcripts (I) and truncated HBV transcripts (II) by RT/PCR comprises a semi-nested PCR using in the second PCR step an upstream primer which is located downstream of the upstream primer of the first PCR step. Nested and semi-nested PCR are well known to the person skilled and can be carried out, e.g., as described in Example 1(D), below, and Figure 1. The sensitivity of nested PCR is known to be about 1000 times higher than the corresponding single round PCR (Cacciola et al., N Engl. J. Med. (1999), 341: 22-26). Its reliability can be guaranteed by well organized procedures and step by step protocols as described by Kwok and Higuchi (Nature (1989), 339: 237-238). In principle the PCR procedures can be automatized in assay systems which combine amplification and proof of specificity. In these systems oligonucletides with fluorescent dyes at opposite ends provide a quenched probe system for the detection of PCR products (e.g. Livak et al. (1995), PCR Meth. and Appl. 4, 357-362). This can be done in automatized fluorescence emission spectra.

The amplified products can be analysed by standard procedures, preferably by agarose gel electrophoresis. The separated PCR products can be further analysed by staining with ethidium bromide or by Southern blot analyses using labelled probes.

The invention is further described with regard to the figures which show:

### Figure 1: Location of primers in relation of HBx DNA and RNA

Numerals indicate positions on viral DNA (Xho I coordinates). Primers are symbolized by arrows carrying the respective designations used in the text. Amplification products are depicted with respective sizes in brackets. CAUAAA and UAUAAA are the signals at which truncated RNA (trRNA) and full-length RNA (fRNA) are polyadenylated. GCUUC(A)ₙ and CCAGC(A)ₙ symbolize the targets for the anchored oligo(dT) primers (see Example 1).

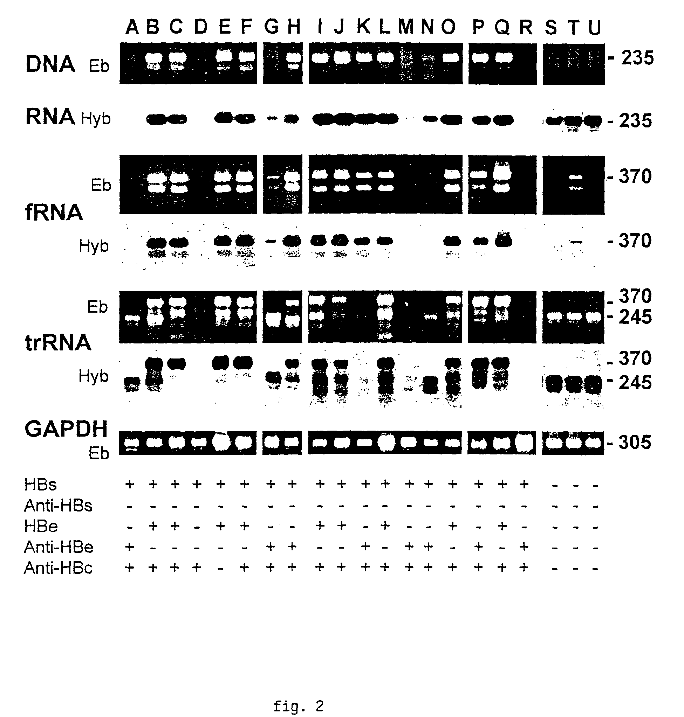

### Figure 2: Representative PCR data obtained on viral nucleic acids from sera

Capital letters at the top indicate individual patients referred to in the specification. Ethidium bromide-stained (Eb); visualized by hybridization (Hyb); glyceraldehyde 3-phosphate dehydrogenase (GAPDH). Expected positions for amplification products of HBx-region DNA and RNA, full-length RNA, truncated RNA, and of GAPDH RNA are indicated together with their sizes (235, 370, 245, and 305 bp, respectively). Amplification products A-H were analyzed within one series (see Examples), I-O, P-R and S-U within respective three others. Corresponding serological data are indicated at the bottom.

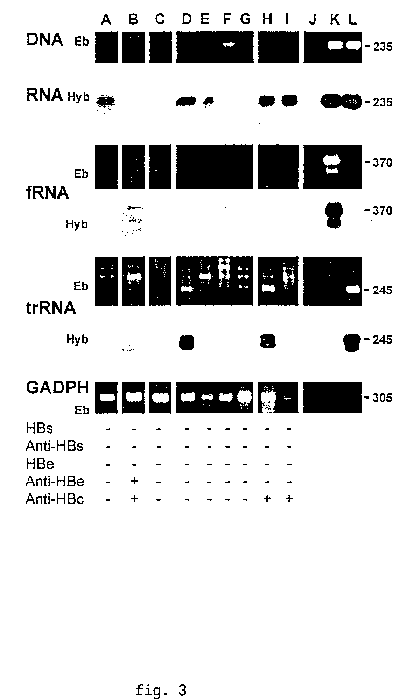

### Figure 3: PCR data on viral nucleic acids from sera of HCV-carriers bearing HCC

Capital letters on the top indicates patients (A-I) and controls: water (J), full-length cDNA pMT9T40A (K) and truncated cDNA pMT9T41A (L). The amplification products of A, B and C, D-L were analyzed within three series. Corresponding serological data are indicated at the bottom. See also legend to Figure 2.

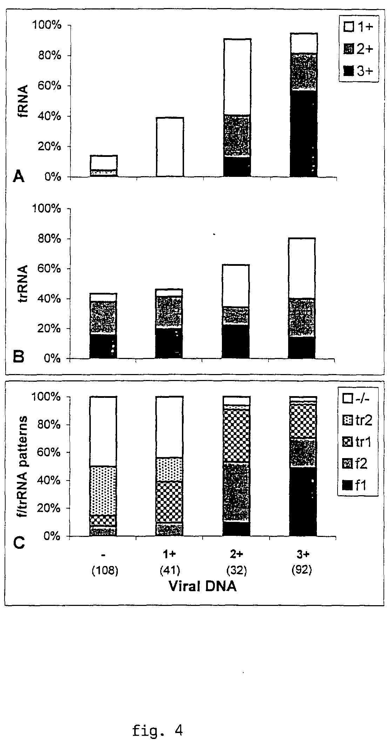

### Figure 4: Serum DNA levels and the representation of full-length and truncated RNA

The fraction of cases positive for full-length RNA (fRNA; A) and for truncated RNA (trRNA; B) are given for groups of patients without (-) or with low (+), moderate (2+) and high levels (3+) of serum DNA. The respective amounts of RNA are indicated by shadings (top right). In panel C, discernible full-length/truncated RNA representation (f/trRNA) patterns are indicated for the same patient groups: absence of both RNA types (-/-), truncated RNA-only (tr2), truncated RNA-dominance (tr1), full-length RNA-dominance at low (f2) and high (f1) levels. Case numbers for each patient groups within brackets are at the bottom.

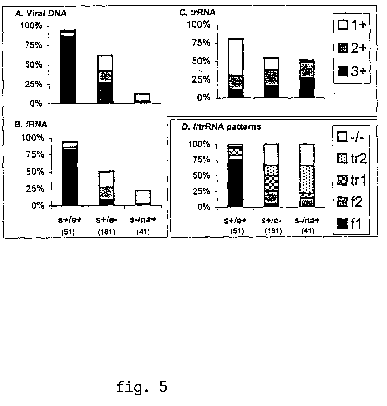

### Figure 5: Representation of viral nucleic acids at HBe and HBs seroconversion

Fraction of cases to different degrees positive for viral DNA (DNA; A), full-length RNA (fRNA; B) and truncated RNA (trRNA; C) in patient groups positive for HBs before (s+/e+) and after (s+/e-) HBe seroconversion, as well as in a group negative for HBs but carrying viral nucleic acids (s-/na+). In panel D, respective full-length/truncated RNA representation (f/trRNA) patterns, as addressed in the legend for Figure 4. Case numbers for individual patient groups are given within brackets at the bottom.

### Figure 6: Viral nucleic acids in sera with different alanine transaminase (ALT) levels

Fraction of cases to different degrees positive for viral DNA (DNA; A), full-length RNA (fRNA; B) and truncated RNA (trRNA; C) in patient groups with distinct ranges of ALT values (IU/L). Case numbers for individual patient groups within brackets are given at the bottom.

### Figure 7: Age-related changes in the representation of viral nucleic acids in sera

Fraction of cases to different degrees positive for viral DNA (DNA; A), full-length RNA (fRNA; B) and truncated RNA (trRNA; C) in groups of patients with increasing ages (up to 10, between 10 and 20 years, etc.). In panel D, respective full-length/truncated RNA representation (f/trRNA) patterns, as addressed in the legend for Figure 4. Case numbers for individual patient groups are given within brackets at the bottom.

The following examples illustrate the invention.

### EXAMPLE 1: General methods

### (A) Serum Samples and Extraction of Nucleic Acids

As shown in Table 1, a total number of 319 cases of sera were used. These included 101 sera, 64 positive and 37 negative for HBs, from HCC patients (71 from Tangdu Hospital in Xi'an, China, 25 from Tianjin Cancer Hospital in Tianjin, China, eight from Shaanxi Cancer Hospital in Xi'an and seven from Takegoshi Clinic in Toyama, Japan), 168 sera from chronic HBs-carriers without a detectable HCC (167 from Tangdu Hospital and one from Takegoshi Clinic) and 50 sera from HBs-negative individuals without any evidence indicating a chronic liver disease (all from Tangdu Hospital). The latter cases were intended to be used as a reference group, including 22 samples from patients with no history of hepatitis or cirrhosis coming mostly (17/22) from countryside and hospitalized for other diseases (5 for glioma, 4 for meningeoma, 10 for lung cancer and 3 for cardiovascular diseases) and 28 samples from apparently healthy local urban inhabitants receiving regular physical and laboratory check-ups. The study protocol was approved by the Medical Ethics Commission of The Fourth Military Medical University. All serum samples were separated as soon as the blood was coagulated, and stored at -80°C in RNase-free Eppendorf tubes. Isolation of nucleic acids was performed using a nucleic acid extraction kit (Roche Diagnostics (former Boehringer Mannheim), Mannheim, Germany) principally following the manufacturer's instructions, with the supplied poly(A) RNA replaced by the same amount of 16S and 22S ribosomal RNA (Roche Diagnostics) as a carrier. For each isolation, 200 *µ*l of serum was used, with 50 *µ*l of extract eluted.

### Upstream primers include:

txs3 (1434): TCT CAT CTG CCG GAC CGT GT
txs1 (1454): GCA CTT CGC TTC ACC TCT GC
txs (1445): GGA CCG TGT GCA CTT CGC TT
GAPDH1 (3755): CAT CTC TGC CCC CTC TGC TGA

### Downstream primers include:

txas5 (1683):    (T)15 GCT GG
Rxas2 (1808):    (T)15 GAA GC
Rxas4 (1806):    (T)15 AGC TC
xas1 (1668):     AAT TTA TGC CTA CAG CCT CC
GAPDH2 (4344):   GGA TGA CCT TGC CCA CAG CCT

### (B) PCR for viral DNA

Two *µ*l of the nucleic acid extract was used for amplification of viral DNA in a reaction volume of 50 *µ*l containing 1.5 mM MgCl₂, 1x PCR reaction buffer (Gibco BRL, Life Technologies Inc, Gaithersburg, MD), 75 ng of each primer (txs3 and xasl; see Fig. 1), 0.2 mM of each dNTP and 1.25 U Taq DNA polymerase (Gibco BRL). 35 cycles were performed as described by Kairat et al., Intervirology 42 (1999), 228-237, (40 s denaturation at 90°C, 50 s annealing at 53°C, and 40 s elongation at 70°C). Amplification efficacy and specificity were demonstrated by including positive controls, using plasmids pMT9T40A (200 pg) and pMT9T41A (200 pg) carrying cloned cDNAs of full-length and truncated HBV transcripts, respectively (Hilger et al., J. Virol. 65 (1991), 4284-4291), and a negative control with water.

### (C) RT-PCR for X-region RNA

All viral RNA molecules spanning the X region, including the full-length and truncated transcripts as well as those polyadenylated neither at the standard nor at the cryptic terminating sites, were collectively designated as HBx RNA. The fraction of HBx mRNA among these molecules may vary (see below). HBx RNA was detected through an anchored RT/PCR procedure following pretreatment of nucleic acid extracts with DNase I. The nucleic acid extracts, 10 *µ*l for each, were digested in a 20 *µ*l of reaction volume containing DNase I (Amplification Grade, Gibco BRL) for 30 min at 25°C. Five *µ*l of the digestion product were applied to the RT-PCR using a one-tube system (Roche Diagnostics) in a reaction volume of 50 *µ*l containing 0.2 mM of each dNTP (Gibco BRL), 5 mM dithiothreitol, 1 x RT-PCR buffer with Mg²⁺, 150 ng of each primer (txs3 and xasl; see Fig. 1) and 1 *µ*l of enzyme mix composed of the high-fidelity enzyme mixture and AMV reverse transcriptase. 35 cycles were performed as described in the reaction for viral DNA after RT (20 min at 50°C). Samples containing cDNA of plasmids PMT9T40A and PMT9T41A, before and after digestion with DNase I, were used as positive controls and to show the digestion efficacy, respectively, with water as a negative control. Glyceraldehyde 3-phosphate dehydrogenase (GAPDH) transcript was also demonstrated via the same RT/PCR procedures to show presence and integrity of RNAs in the sera using the primers GAPDH1 and GAPDH2 as described with tissue samples (Hsu et al., Int. J. Cancer 55 (1993), 397-401).

### (D) Demonstration of full-length and truncated HBV transcripts via a semi-nested PCR following RT

As anchored oligo(dT) primers were involved throughout the reactions for full-lenth and for truncated transcripts (see Fig. 1), pretreatment of samples with DNase I was not necessary. Nucleic acid extracts, 10 *µ*l for each, were used for the first-round PCR (anchored RT-PCR) with a one-tube system in a reaction volume of 50 *µ*l as described above in the reaction for HBx RNA. An amount of 150 ng of the upstream primer (txs3 for full-length and txs for truncated RNA) and 300 ng of the downstream anchored oligo(dT) primer (Rxas2 and Rxas4 for full-length and txas5 for truncated RNA) were added for each reaction. The amplification products, two *µ*l for each, were then subjected to the second-round PCR as described for the viral DNA amplification, with txs1 as the upstream inner primer in both reactions for full-length and truncated transcripts, and the same downstream anchored oligo(dT) primers as in the first-round PCR for each viral transcripts.

Efficacy and specificity of the reaction for the full-length transcript were demonstrated, for each series of samples, by including plasmid pMT9T40A (50 pg) as a positive control, and plasmid pMT9T41A (50 pg) and water as negative controls, as described previously (Kairat et al., Intervirology 42 (1999), 228-237). As for the amplification for the truncated transcript, plasmid pMT9T41A was used as a positive control, and plasmid pMT9T40A and water as negative controls. In addition, an RNA extract from an HBV-infected liver, shown previously to contain only the full-length transcript (Kairat et al., Intervirology 42 (1999), 228-237), and another extract of HCC tissue from an HBV-infected patient, shown to contain only the truncated transcript (Kairat et al., Intervirology 42 (1999), 228-237), were also used as a positive control in the reactions for the full-length and truncated viral transcripts, respectively.

### (E) Probe Preparation

A DNA probe for hybridization was prepared through PCR with digoxigenin (Dig)-11-dUTP (Roche Diagnostics), with pMT9T40A as a template and txsl and xasl as its upstream and downstream primers, respectively. A low-incorporation ratio (Dig-11-dUTP:dTTP=1:20) was used. It was intended to verify visible DNA products as HBx-specific rather than increasing the sensitivity of the detection-procedure. After purification through ethanol precipitation, the incorporation efficacy was estimated by dot immunoassay using a Dig-labeled control DNA (Roche Diagnostics) diluted serially according to the instructions of the manufacturer.

### (F) Electrophoresis, Southern Blotting and Hybridization

Amplification products, 15 *µ*l for each, were separated on a 2% agarose gel in the presence of ethidium bromide (0.2 *µ*g/ml) and visualized under UV light. For the viral transcripts and DNA, agarose gels were blotted onto Hybond-N⁺ nylon membranes (Amersham, Buckinghamshire, England). The PCR products were then hybridized with the Dig-labeled DNA probe. Hybridization signals were demonstrated by immunoreactions through the consecutive application of a sheep antibody against Dig (Roche Diagnostics) and alkaline phosphatase-labeled rabbit anti-sheep IgG (Jackson ImmunoResearch Laboratories, Inc., West Grove, PA) and finally visualized with nitroblue/5-bromo-4-chloro-3-indolyl phosphate solution for about 15 min as described previously (Su et al., Hepatology 27 (1998), 1109-1120).

### (G) Statistical Analysis

Positive signals for the viral transcripts and DNA were evaluated in reference to their expected sizes, their intensities being graded as strong (3+), moderate (2+) and weak (1+), representing high, moderate and low levels, respectively, regarding contents of the corresponding target elements measured. Possible associations of full-length and truncated viral transcripts to the age of the donors, hepatitis Be antigen (HBe) status, viral DNA level and levels of alanine transaminase (ALT) values were investigated using graphical and non-parametrical statistical methods. P < .05 was regarded as a significant difference.

### EXAMPLE 2: Serodetection of viral transcripts and DNA

Nucleic acids extracted from sera of patients positive or negative for HBs and with or without HCC were analyzed for viral DNA and RNA with homology to the HBV X-gene region. Primarily it was examined whether the comparatively simple approach using sera could reveal similar distinct transcription and polyadenylation patterns as observed previously for RNA extracted from tissue samples.

Figure 1 outlines the following individual HBx-region-specific PCR analyses which were carried out on nucleic acids from sera: (1) a PCR for the detection of DNA carrying HBx sequences between map positions 1434 and 1668, as indicated at the top; (2) an RT/PCR for detection of RNA spanning the same sequence as for viral DNA; (3) an RT/PCR recognizing the junction structure at the poly(A) addition site of full length transcripts [GCUUC(A)ₙ]; and (4) a similar RT/PCR with anchored oligo(dT) primers recognizing the corresponding structure on truncated transcripts [CCAGC(A)ₙ]. The anchored oligo(dT) primers for full-length RNA first were used in conjunction with a sense primer starting at position 1434 and the one for truncated RNA in conjunction with a sense primer starting at position 1445. In subsequent second-round PCR the same sense primer starting at position 1454 was used for both assays. A Dig-labeled probe, an amplification product spanning map positions 1454 and 1668, was used to confirm the HBx-sequence specificity of amplification products displaying the predicted sizes.

Presence and integrity of cellular RNA in nucleic acid extracts were proven via amplification for GAPDH RNA sequences. For all 319 sera examined, a product of 305 bp was obtained, as expected for GAPDH transcripts (Hsu et al., Int. J. Cancer 55 (1993), 397-401), but not that of 590 bp, the amplification fragment expected for its DNA template.

Special attention was paid to avoid contamination of the samples, as described by Kwok and Higuchi, Nature (London) 339 (1989), 237-238. The reliability of the reactions was ensured by including corresponding positive and negative controls in test series of about 15 samples. The inclusion of positive controls also allowed to compare signal intensities at comparable staining conditions. Assays were carried out at least twice in independent series. In the few cases in which unexpected results were obtained for the controls, the tests were repeated for the whole series until reproducibility was obtained.

Results from the reactions, for representative cases, are shown in Figures 2 and 3 to demonstrate the clear differences in the individual HBx signal patterns. For the amplification products of viral DNA and GAPDH mRNA only the ethidium bromide-stained gels are shown. For the amplification products of HBx RNA the hybridization data are shown, for full-length and truncated RNA, in addition the ethidium bromide-stained gels are shown. Signals for HBx RNA irrespective of polyadenylation (Figs. 2 and 3, RNA) are present in most of the cases selected but displayed a wide range of intensities. The signal for full length RNA visualized both by ethidium bromide staining and by hybridization appears as a double band, with a stronger signal for the amplification product migrating at the expected position of 370 bp DNA and a less intensive band for faster migrating material most probably being single stranded plus-strand DNA produced during an asymmetric amplification. In all cases positive for the full-length transcripts, the amplification of the 3'-end region of truncated RNA gave rise to a 370 bp amplification product as obtained in the reaction for full-length RNA and to the 245 bp product expected for truncated transcripts. Presence of the 370 bp product in the assay for truncated RNA was consistent with that demonstrated in the assay for the full-length transcripts, but presence of the 245 bp product in the former assay appeared to be independent of the presence or absence of the 370 bp product in the latter assay (Fig. 2, compare cases A, B and C). Apparently the RT/PCR procedure adapted here for the analysis of truncated RNA from sera recognized both full-length and truncated RNA.

Positive signals were graded into weak (+), moderate (2+) and strong (3+) intensities, and related to signal intensities obtained on 1 fg, 10-100 fg and 1 pg or more, respectively, of cDNA (pMT9T41A). This estimation did not take into account the influence of background RNA. The contents of target elements in nucleic acids extracted from sera then are minimum estimates.

Based on presence and intensities of signals for full-length and truncated transcripts, five patterns were differentiated, namely full-length RNA-dominance at a high (3+; Fig. 2, cases B, C, E, F, H-J, O and Q) and lower levels (2+ or 1+; Fig. 2, case K and Fig. 3, case B), truncated RNA-dominance (Fig. 2, cases G and T), truncated RNA-only (ranging from 1+ to 3+; Fig. 2, cases A, M, N, S and U, and Fig. 3, cases D and H) and absence of both RNA types (-/-; Fig. 2, case D and R, and Fig. 3, cases A, E and I).

### EXAMPLE 3: Viral transcripts in HBs-positive sera

Signals for HBV transcripts were identified in the majority of the 232 HBs-positive samples (Table 1). Overall the full-length and truncated transcripts were detected in a similar frequency of about 60%, but their representations followed different patterns. No significant difference was observed regarding the prevalence of truncated transcripts in sera from HCC-bearing (52.8%) and those from HCC-free (55.9%) patients with their ages ranging between 31 and 70 years (p>0.05). However, there was a significant difference in the prevalence of full-length transcripts between these two groups, with that for HCC-bearing (62.5%) being higher than that for the HCC-free (47.06%) cases (p). In line with this observation a higher level of viral DNA was found in sera from HCC patients (70.8 % vs. 50.0 %, p).

HBx-region RNA was nearly always (169/170) present when full-length and/or truncated viral transcripts were present. The signal intensities were in accordance with those for full-length transcripts, if present (Fig. 2). In truncated transcript-dominance or patterns where only truncated RNA was present, signals for HBx RNA, detected using one round of PCR following RT, often appeared weaker than that for truncated transcripts obtained following the semi-nested (double-round) PCR procedure (Fig. 2, cases A, G, N and S). As listed in Table 1, there were still some samples from HBs-positive series (62/232, 26.7%), in which neither the full-length nor the truncated transcript was identified. In 39 (62.9%) of these cases, a positive signal was obtained only by the assay for HBx RNA (Fig. 2, case D), indicating the presence of transcripts polyadenylated at neither of the two viral poly(A) signals.

### EXAMPLE 4: Full-leng, but not truncated, transcripts in sera are related to replicative processes

As listed in Table 1, the sera from 41 individuals without detectable circulating HBs were shown to contain some viral nucleic acids (see below). Therefore, they are here also considered as chronic HBV-carriers. Both HBs- (232 cases) and the viral nucleic acid-carriers (41 cases) were subjected to Mann-Whitney analysis to explore the relationship between the level of the full-length or truncated viral transcript and that of viral DNA as an indicator of replication. As shown in Fig. 4 A, a close correlation was found between the levels of full-length transcripts and viral DNA (p). The fractions of full-length RNA-dominant patterns at high and lower levels dropped along with viral DNA levels. Sera with high-level full-length transcripts were nearly always found to carry high content of viral DNA. In contrast, no significant association was observed in overall levels between truncated transcripts and circulating viral DNA (Fig. 4B). The truncated RNA-only pattern appeared to be present predominantly in the absence of viral DNA (Fig. 4C). The presence of circulating truncated RNA thus was independent of a marker indicative for replicative processes.

### EXAMPLE 5: HBe and HBs seroconversion is correlated with the decline of full-fength transcripts

The content of viral DNA in sera with HBe was shown to be high for most samples tested, being reduced greatly in majority of the cases after HBe seroconversion irrespective of whether the sera contained anti-HBe or not. It was absent in most HBs-negative sera (Fig. 5A). A similar pattern was also observed for full-length viral transcripts (Fig. 5B). The average ages of the HBe-positive and -negative HBs-carriers and the HBs-negative carriers were 28,7 (HBe-positive), 42,0 (HBe-negative) and 52,7 (HBs-negative). Their difference being significant (p). It can be concluded that there is a sequential change in the ratio of the two forms of viral transcripts starting with cases with full-length RNA-dominance at high level proceeding to cases with truncated RNA-dominant or truncated RNA-only patterns (Fig. 5 B-D). Again the presence in sera of truncated RNA appeared to be independent of replicative processes (Fig. 5C).

### EXAMPLE 6: Viral DNA and full-length transcripts are related to alanine transaminase (ALT) levels

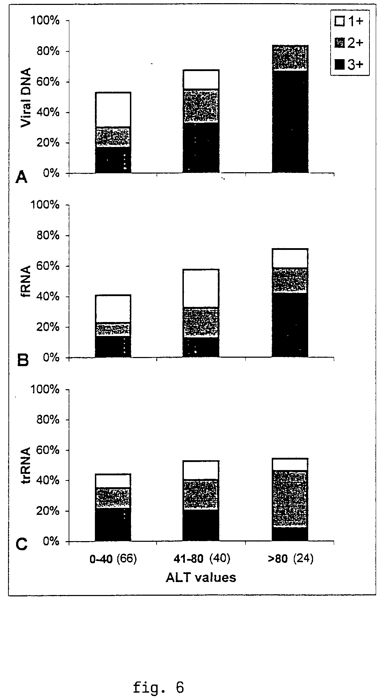

It was conceivable that the presence of the viral RNA in sera is related to the damage to hepatocytes. If so, the assay system employed in this invention would only be of relevance for patients with a chronic hepatitis of measurable activity. Serum ALT values, whose elevation is believed to reflect liver damage, then would be expected to be correlative with the viral transcripts in circulation. To study this problem, 130 HBs-positive cases, whose serum ALT values were available, were divided into groups with normal range (0-40 IU/L) and with slight (41-80 IU/L) or marked (> 80 IU/L) elevation. The fraction of the full-length transcripts was shown to be increased along with the elevation of ALT value (Fig. 6B), in parallel with the levels of the viral DNA (Fig. 6A). However, for the levels of the truncated transcripts no significant correlation to ALT values was observed (Fig. 6C). Hence, the presence of viral transcripts is not necessarily related to liver damage. The correlation observed between viral DNA and full-length RNA and ALT levels (Fig. A and B) simply reflects liver damage due to replication.

### EXAMPLE 7: Age-related progression to non-replicative stages and the persistence of truncated RNA

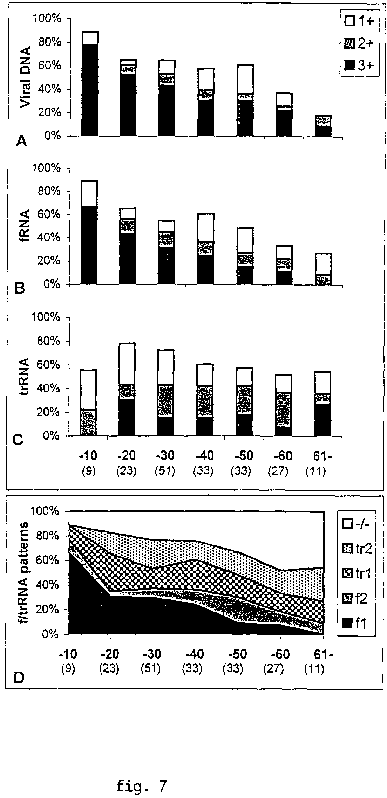

As most of the chronic carriers in endemic areas are believed to be infected from their neonatal stage or early childhood, viral RNA and DNA patterns in sera from 168 HBs-positive and 19 viral nucleic acid-positive carriers of different ages were compared to explore their changes in the course of the infection. Data from sera of patients with HCC were excluded from this analysis. At young age, the full-length transcripts were found at high levels and dropped in its representation in the latter decades of the infection. This appeared to be in good agreement with age-related decline of the viral DNA, but it was more pronounced for full-length RNA than for viral DNA (Fig. 7, A and B). A significant age-dependent change for the presence of the truncated transcript in sera was not found. Its representation was much lower as compared to those of the full-length transcript in the first decade (p), reached a peak at the second decade to remain at high levels for the following decades (Fig. 7C). As shown in Fig. 7D, this resulted in the fast reduction of cases with full-length RNA-dominance and a corresponding increase with truncated RNA-dominance or -only patterns in the second decade of life. For the cases at ages from 30 to 50 years, full-length RNA-dominance at a high level gradually gave space to the dominance at lower levels. This transition to lower levels of full-length RNA was found mainly in the cases seronegative for HBe, whose viral DNA level remained high (Fig. 4A, right column; Fig. 5 A and B, middle columns). The mean ages for the cases with full-length RNA-dominance at a high level (25.7) and lower (37.7) levels, truncated RNA-dominance (34.6), truncated RNA-only (40.0) and absence of both RNA types (41.0) align consecutive hepatitis stages with overlap at the transition from full-length to truncated RNA-dominance. It can be concluded that the age-dependent changes in viral RNA patterns reported earlier based on data gained on liver tissue similarly can be observed via serum assays.

### EXAMPLE 8: Viral transcripts in HBs-negative sera from HCC patients

HBV transcripts and DNA were also detected in HBs-negative samples from HCC patients at a high frequency (22/37, 59.5%), the prevalence being significantly higher in sera positive (11/14, 78.6%) than in those negative (11/23, 47.8%) for anti-HBc (p < 0,09). Among the 22 positive cases, 17 were positive only for viral transcripts, 3 both for the transcripts and for viral DNA and 2 only showing a weak signal for viral DNA. Truncated viral transcripts were detected more frequently (10/37, 27.0%; p) than full-length transcripts (7/37, 18.9%), with HBx RNA identified by the HBx RNA assay in exceeding 50% of the cases (20/37; Table 1).

Among the HBs-negative sera, nine were from hepatitis C virus (HCV)-positive HCC patients (six from Japan and three from China). Seven of them were shown to be positive for the HBV viral nucleic acids tested. Five cases showed positive signals via the HBx RNA assay, two of them being also positive for truncated transcripts. In one case only full-length transcripts and in another only viral DNA were found (Fig. 3). Taken together, the data support the view that the involvement of HBV in hepatocarcinogenesis, as evaluated through serodetection for HBs, may be underestimated.

### EXAMPLE 9: Viral transcripts in HBs-negative sera from individuals without liver diseases

HBs-negative sera from 50 individuals free of HCC, 22 from patients without any evidence indicating a liver disease and 28 from apparently healthy individuals, were also examined. Viral transcripts were found in 19 sera (38 %). In no case viral DNA was detected (Table 1 and Fig. 2, cases S-U). Among the 19 positive cases, 11 were for transcripts polyadenylated exclusively (nine cases) or predominantly (two cases) at the cryptic poly(A) signal, eight only for transcripts polyadenylated at neither of the two viral poly(A) signals, i.e. transcripts detected by the assay for total HBx RNA.

### Annex to the description documents - subsequently filed sequences listing

SEQUENCE LISTING

<110> Deutsches Krebsforschungszentrum

<120> Method of diagnosing HBV infection stages

<130> K 2811

<140> EP 00109436.6
<141> 2000-05-03

<160> 9

<170> PatentIn Ver 2.1.

<210> 1
<211> 20
<212> DNA
<213> Artificial Sequence

<220>
<223> Description of the Artificial Sequence:
Primer

<400> 1

tctcatctgc cggaccgtgt

<210> 2
<211> 20
<212> DNA
<213> Artificial Sequence

<220>
<223> Description of the Artificial Sequence:
Primer

<400> 2

gcacttcgct tcacctctgc

<210> 3
<211> 20
<212> DNA
<213> Artificial Sequence

<220>
<223> Description of the Artificial Sequence:
Primer

<400> 3

ggaccgtgtg cacttcgctt

<210> 4
<211> 21
<212> DNA
<213> Artificial Sequence

<220>
<223> Description of the Artificial Sequence:
Primer

<400> 4

catctctgcc ccctctgctg a

<210> 5
<211> 20
<212> DNA
<213> Artificial Sequence

<220>
<223> Description of the Artificial Sequence:
Primer

<400> 5

tttttttttt tttttgctgg

<210> 6
<211> 20
<212> DNA
<213> Artificial Sequence

<220>
<223> Description of the Artificial Sequence:
Primer

<400> 6

tttttttttt tttttgaagc

<210> 7
<211> 20
<212> DNA
<213> Artificial Sequence

<220>
<223> Description of the Artificial Sequence:
Primer

<400>	7

tttttttttt tttttagctc

<210>	8
<211>	20
<212>	DNA
<213>	Artificial Sequence

<220>
<223>	Description of the Artificial Sequence:
	Primer

<400>	8

aatttatgcc tacagcctcc

<210>	9
<211>	21
<212>	DNA
<213>	Artificial Sequence

<220>
<223>	Description of the Artificial Sequence:
	Primer

<400>	9

ggatgacctt gcccacagcct

**Table 1.**

| **Prevalence of HBV transcripts and DNA in sera positive or negative for HBs from individuals with or without HCC** | | | | | | |
|---|---|---|---|---|---|---|
| | **Cases** | **Cases** | **Viral** | **Viral transcripts (%)** | | |
| **Sera donors** | **tested** | **positive (%)** | **DNA (%)** | **HBx RNA** | **fRNA** | **trRNA** |
| HBs+ | 232 | 215 (92.7) | 160 (69.0) | 208 (89.7) | 139 (59.9) | 139 (59.9) |
| HCC-bearing | 64 | 57 (89.1) | 53 (82.8) | 53 (82.8) | 42 (65.6) | 31 (48.4) |
| HCC-free | 168 | 158 (94.0) | 107 (63.7) | 155 (92.3) | 97 (57.7) | 108 (64.3) |
| HBs-/HCC-bearing^{a} | 37 | 22 (59.5) | 5 (13.5) | 19 (51.4) | 7 (18.9) | 10 (27.0) |
| anti-HBc+ | 14 | 11 (78.6) | 2 (14.3) | 9 (64.3) | 4 (28.6) | 6 (42.9) |
| anti-HBc- | 23 | 11 (47.8) | 3 (13.0) | 10 (43.5) | 3 (13.0) | 4 (17.4) |
| HBs-/HCC-free ^{b} | 50 | 19 (38.0) | 0 (0.0) | 19 (38.0) | 2 (4.0) | 11 (22.0) |
| Other patients ^{c} | 22 | 14 (63.6) | 0 (0.0) | 14 (63.6) | 2 (9.1) | 10 (45.5) |
| Healthy individuals ^{d} | 28 | 5 (17.9) | 0 (0.0) | 5 (17.9) | 0 (0.0) | 1 (3.6) |

| | | | | | | |
|---|---|---|---|---|---|---|
| Abbreviations: fRNA, full-length viral RNA; HBc, hepatitis B core antigen; HBs, hepatitis B surface antigen; HBV, hepatitis B virus; HBx, HBV x protein; HCC, hepatocellular carcinoma; trRNA, truncated viral RNA; +, seropositive reaction; -, seronegative reaction. ^{a} Thirty samples being from Chinese patients and seven from Japanese patients, six and three positive for anti-HCV among the former and the latter, respectively. | | | | | | |
| ^{b} Thirteen samples being seropositive only for anti-HBs, one only for anti-HBc, and others negative for all HBV seromarkers. | | | | | | |
| ^{c} Nineteen with neoplasms (ten with lung cancers, five with gliomas and four with meningeomas), samples being taken during checkups before their operations, and three w with heart diseases. | | | | | | |
| ^{d} All for routine physical and laboratory checkups. | | | | | | |

## Claims

1. A method for the diagnosis of HBV infection stages which comprises the identification of full-length HBV transcripts (I) and truncated HBV transcripts (II) in a serum sample wherein the ratio of I:II is indicative of a particular infection stage.

2. The method of claim 1 wherein a ratio of I:II > 1 is indicative of a highly replicative HBV infection stage.

3. The method of claim 1 wherein a ratio of I:II < 1 is indicative of a chronic HBV infection stage.

4. The method of claim 1 or 3 wherein a ratio of I:II < 1 is indicative of a risk to develop HCC.

5. The method of claim 1 wherein the absence of full-length HBV transcripts (I), i.e. ratio of I:II < 1, is indicative of occult stages of HBV infection.

6. The method of any one of claims 1 to 5 wherein the HBV transcipts are transcripts comprising the X-region of HBV (HBx RNA).

7. The method of any one of claims 1 to 6 wherein identification of full-length transcripts (I) and truncated HBV transcripts (II) is carried out by RT/PCR using downstream primer(s) allowing to discriminate between full-length HBV transcripts (I) and truncated HBV transcripts (II).

8. The method of claim 7 wherein the downstream primers are oligo(dT)anchored primers.

9. The method of claim 8 wherein the oligo(dT)anchored primers comprise the nucleotide sequence (T)₁₅ GCT GG, (T)₁₅ GAA GC and/or (T)₁₅ AGC TC.

10. The method of any one of claims 7 to 9 wherein the identification of full-length HBV transcripts (I) and truncated HBV transcripts (II) by RT/PCR comprises a semi-nested PCR using in the second PCR step an upstream primer which is located downstream of the upstream primer of the first PCR step.

11. The method of any one of claims 7 to 10 wherein the PCR-products are **characterized by** agarose gel electrophoresis or automatized fluorescence emission spectra.

12. Kit for carrying out the method of any of claims 1-11 comprising at least one of the oligo(dT)anchored primers of claim 9.
